# EUROPEAN PATENT SPECIFICATION

(11) **EP 1 809 165 B1**
(45) Date of publication and mention of the grant of the patent: **28.06.2017**
(21) Application number: 05790075.5
(22) Date of filing: 03.10.2005
(51) Int. Cl.: A61B 5/00, A61B 5/103

(54) **VIBROTACTILE PERCEPTION METER**
VIBROTAKTILER WAHRNEHMUNGSMESSER
DISPOSITIF DE MESURE DE PERCEPTION VIBROTACTILE

(30) Priority: 25.10.2004 SE 0402569
(43) Date of publication of application: 25.07.2007
(73) Proprietor: Vibrosense Dynamics AB, 205 12 Malmö (SE)
(72) Inventor: SPEIDEL, Antonio, 230 42 Tygelsjö (SE)
(74) Representative: KIPA AB
(86) International application number: PCT/SE2005/001450
(87) International publication number: WO 2006/046901

(56) References cited:
- EP-A2- 0 072 255
- WO-A1-00/59377
- WO-A1-00/59377
- US-A- 4 175 546
- US-A- 5 002 065
- US-A- 5 195 532
- US-A- 5 381 805
- US-A- 5 433 211
- HARADA N. ET AL: 'Factors influencing vibration sense thresholds used to assess occupational exposures to hand transmitted vibration' BRITISH JOURNAL OF INDUSTRIAL MEDICINE vol. 48, no. 3, March 1991, pages 185 - 192, XP008117590
- "Mechanical vibration -- Vibrotactile perception thresholds for the assessment of nerve dysfunction -- Part 1: Methods of measurement at the fingertips", ISO 13091-1:2001,, 1 January 2001 (2001-01-01), XP009152673,
- Roshada Daud ET AL: "A Pilot Study of Reference Vibrotactile Perception Thresholds on the Fingertip Obtained with Malaysian Healthy People Using ISO 13091-1 Equipment", Industrial Health 2004, 42, 189-195, 1 June 2004 (2004-06-01), pages 189-195, XP55008571, Retrieved from the Internet: URL:https://www.ncbi.nlm.nih.gov/pubmed/15 128168 [retrieved on 2011-09-30]

## Description

### Field of invention

This invention relates generally to an apparatus for detecting sensory deficits in peripheral nerves and, in particular, to an apparatus for detecting sensory deficits associated with neuropathy in the fingers, or other parts of the body. The present invention relates to a method and a system for identifying vibrotactile perception thresholds of different mechanoreceptors at a skin site of a subject to assess sensory change in tactile sensory nerve function, and wherein the resultant threshold signals obtained by the method are substantially void of errors or inconsistencies.

### Background of invention

Many provocative tests have long been used to detect the very early sensory impairment which usually occurs in diabetes neuropathy and neuropathy caused by vibration exposure. These tests include e.g. the "two-point discrimination" test for tactile gnosis and the monofilament test for touch/pressure. However, for various reasons, these tests have been found to be ineffective in early stages of neuropathy. On the contrary, the vibration sense of the hand is very early impaired in metabolic or vibration-induced neuropathy and various tests on vibration perception of the hand have therefore been developed. However the effectiveness of the vibratory tests is dependent on many test parameters, such as frequency, dimensions of the vibrating rod or probe area and very important, the probe contact force".

The measurement technique involves supporting the body part containing the skin site to be studied, and stimulating the skin surface with vibration under controlled contact conditions in such a way that a single mechanoreceptor population mediates the threshold at each frequency. Accordingly, it is a principal objective of the present invention to provide a screening or diagnostic system to measure the extent of sensory disturbances in neuropathies or any response to treatment of any such sensory disturbance. These objectives are attained generally, in a system to sense a body pressure sensitivity phenomenon of a patient, that includes a vibratory stimulator to apply controlled and compensated vibratory force to a finger (or other body part) of the patient; a drive mechanism connected to effect vibration of the vibratory stimulator.

In US 5,002,065 (LaCourse et. al.) a method is described on how to achieve a well-controlled amplitude and acceleration on the probe by utilising a closed loop control system. However, this means that the amplitude and acceleration will be independent of the applied contact force on the vibrating probe. The used backpressure monitor measures the acceleration on the vibrating probe without any consideration to the applied contact force, which considerably degrades testing reliability.

In US 5,433,211 (Brammer et. al. the applied contact force on the vibrating probe is controlled by an external complex mechanical counter weight mechanism without actually measuring the applied contact force. This complexity increases the possibility of erroneous test set up conditions without any control of the test environment, i.e. the measured vibrotactile perception thresholds (VPT) may be recorded with incorrect applied contact force without any notice, which degrades testing reliability.

In Patent US 5,673,703, Fisher et. al. describes an apparatus for automatic testing of vibrotactile responses of a patient. In the preferred embodiment of the invention, a general-purpose computer functions to control the operation of the system and to record and store the patient's responses. Indentations and vibrations are produced by off-axis rotation of a stimulation probe. A frequency-modulated signal generated by the computer is used to control a motor, which drives the stimulation probe. This apparatus falls short since changes in the contact force will affect both the motor speed (frequency) and the amplitude for the stimulus probe. In fact the described principle for generating the probe movement will measure vibrotactile perception thresholds (VPT) with a very low precision and accuracy due to the inferior control mechanism and test set up. Thus the detected VPT will strongly vary depending on the applied contact force that is not controlled in any way and, accordingly, degrades testing reliability

In WO0059377A1 (LaCourse et. al.) the applied contact force is measured indirectly by measuring the applied force on a surround at which the finger rests during the test. This method requires more complex test equipment and the required applied contact force is much larger compared to when measuring without any surround. A higher contact force will also require a stronger (larger) vibrator that consumes more power which will further increase both the physical weight and the manufacturing cost for the Device (Instrument).

US 5,195,532 discloses an apparatus mechanically stimulating skin with a vibrating tappet that is mounted on a ferromagnetic pole plate of a vibration actuating unit enclosed in a housing. The apparatus controls the vibrational amplitude of the tappet in response to continuous measurements by an accelerometer on the tappet to control the actuation of the tappet. This makes the amplitude of vibration independent of the force with which the tappet is applied to the skin. A sensor attached to a spring between the housing and the tappet can be used to determine application force and tappet position relative to the housing.

US 4,175,546 discloses a device for measuring the sensitivity of a patient to an externally applied vibration. The device comprises a vibrating probe on a pallometer head supported on a beam and a counterweight so that the probe engages a member of the patient with a controlled pressure.

EP 0 072 255 A2 discloses an apparatus and method for monitoring the cutaneous sensitivity of a person involving a vibrating member placed against the skin. The pressure applied is controlled by placing the probe on a bean with a counter balance.

US 5,381,805 discloses an apparatus for performing multiple tests involving responses to thermal stimuli, pain, touch, and vibration. Probes for delivering stimuli are housed in a dome and an arm rest supports and controls the position of a hand on the dome. The dome is rotated to align probes with locations on a subject's hand and the position of a probe relative to the dome can be determined by a combination of a light emitting diode on the probe and a light sensor on the dome.

Details for measuring vibrotactile perception thresholds are disclosed in Daud et al. (2004) Industrial Health 42:189-195 and "Mechanical vibrationVibrotactile perception thresholds for the assessment of nerve dysfunction -- Part 1 : Methods of measurement at the fingertips" ISO 13091-1 (2001) XP009152673.

### Disclosure of invention

To provide means to control and monitor the static and dynamic contact force between human skin and a vibrating probe. This is very important when measuring the Vibrotactile Perception Thresholds (VPT) in order to get accurate test conditions (set up) to achieve required measurement precision.

### Brief description of the drawings

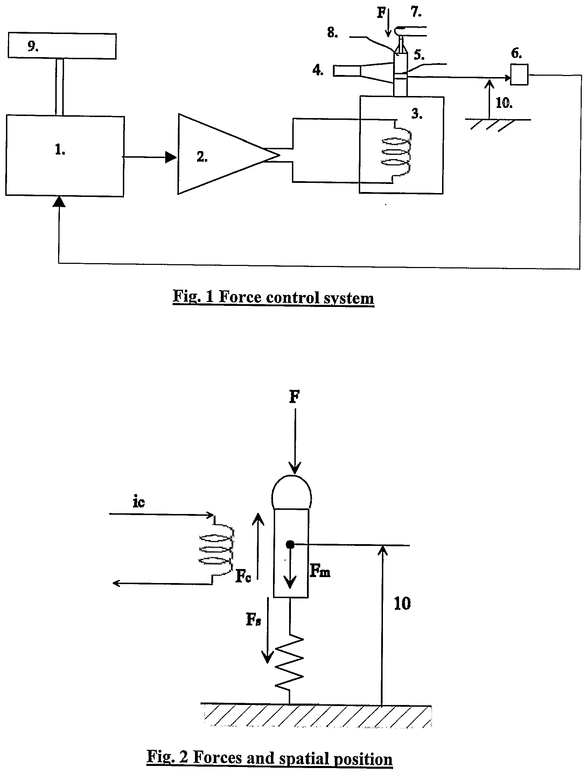

Figure 1; the Force control system which discloses a Micro Computer System (1) comprising a microprocessor and interface electronics with AD- and DA-converters; further is disclosed an amplifier (2) which amplifies an analogue signal from the Micro Computer System (1), the amplified signal drives the Electro Dynamic Vibrator (3). Said vibrator (3) is an electro dynamic device with an attached probe (8), which is moving when a current or a voltage is applied to said device. Transmitter (4); A transmitting device, which sends out some kind of signal. This signal may be an optical beam (light) or an electrical or a magnetic field. Aperture (5); A device, e.g. a hole or a lens, which limits or focuses the transmitted signal in space. The aperture is optional and is not required if the transmitted signal is narrow enough. Detector (6); A device that detects static or a dynamic spatial position of the Vibrating Probe (8) by measuring the transmitted signal from the Transmitter (4) in an appropriate manner. Human skin/body part (7); A human body part, e.g. a finger or a toe, which is pressed with the force F against the Vibrating Probe (8). Vibrating Probe (8); A probe that is fixed attached to the moving part, e.g. a membrane, in the Electro Dynamic Vibrator (3). Human feed back Device (9); A device used by the Micro Computer System (1) to report the displacement in the position caused by the force F. Spatial position (10); Spatial position relative a fixed reference point, origin.
Figure 2; Shows forces and spatial position where i**_{c}** is the current through the Electro Dynamic Vibrator (3), **F_{c}** which is the Probe Force created by the current i_{c} supplied to the Electro Dynamic Vibrator (3), F**ₛ** shows the spring force created by the probe offset inside the Electro Dynamic Vibrator (3), **m** is the moving mass (probe + membrane) in the Electro Dynamic Vibrator (3), **Fₘ** which is the gravitation force on the moving mass (m), **F** is the external force caused either by a calibration force (weight) or by a pressure from a body part and x which is the spatial position (10) relative a fixed reference point, origin.

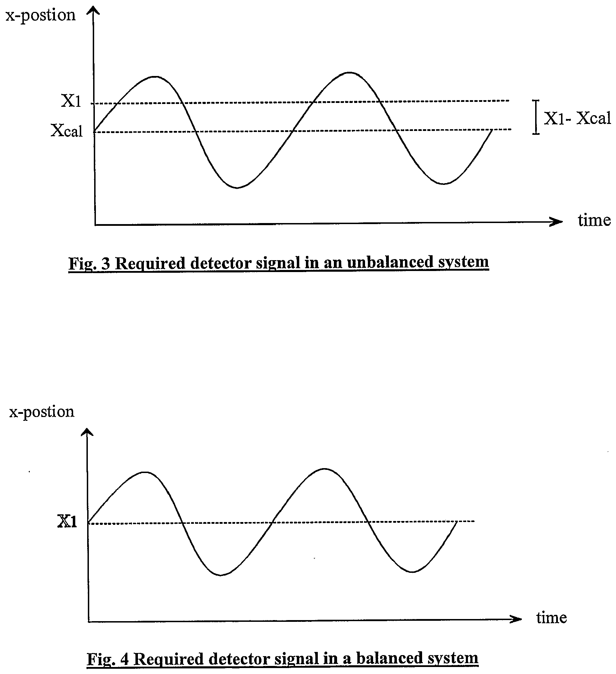

Figure 3; Shows the Required detector signal in an unbalanced system where **x₁** is the spatial position (10) for an unloaded system (x₁) and **x_{cal}** which is the spatial position (10) when the calibration weight is mounted on the vibrating probe (8) without added DC-current (i_{c}), i.e. when i_{c}=0
Figure 4; Shows the required detector signal in a balanced system

### Detailed description of the invention

### Definitions, Abbreviations and acronyms

In the context of the present application and invention the following definitions apply:
- VPT: Vibrotactile Perception Threshold
- RFD: Requested Force Displacement
- RF: Requested Force
- CF: Contact Force
- SI: Sensibility Index
- rms: Root Mean Square

### Description

The invention is a screening or diagnostic testing apparatus, a system and a method of said screening for peripheral neuropathies. In its most basic form, the apparatus includes a surface having an opening, a surround disposed around the opening for a vibrating rod, disposed within said opening for contact with the pulp of a finger or other body part. The preferred apparatus includes a pressure sensor for sensing a pressure exerted by the body part upon the probe to ensure that pressure applied to the body part is within a specified range, means for ensuring said continuous contact with the body part.

Detection principle; the contact force (F) is created by the patient by pressing the body part to be examined (7) against the vibrating probe (8). The patient controls the applied force (F) by adjusting the force (F) in accordance to the reading on the output presented by the "Human feed back Device" (9). The correct force (F) is then applied when the "Human feed back Device" (9) presents a predetermined condition, e.g. correct colour, sound, numerical value etc.

The applied force (F) is measured indirectly by measuring the change of the spatial position (10) on the vibrating probe as a static displacement caused by the force (F). Since the vibrating probe is mounted in a spring supported mechanical construction, any displacement corresponds to a specific force in a linear fashion. Therefore the displacement will be an indirect measure of the applied force, i.e. the force (F) may be calculated by measuring the occurred static displacement of the spatial position (10).

The unloaded Electro dynamic Vibrator (3), i.e. without an applied external force (F) may be calibrated by adding a well-known force, the Requested force (RF), e.g. a "calibration weight". The occurred displacement for the unloaded Electro dynamic Vibrator (3), i.e. the difference in the spatial position (10) of the probe with and without the "calibration weight" will then correspond to a specific force.

The displacement caused by the "calibration weight" is denoted as the "Requested Force Displacement" (RFD). During normal operation the RFD may be used as a requested absolute static offset which should be maintained during the complete test cycle.

A contact Force (F) below or above the RF will be presented on the "Human feed back Device" (9) as a "too low value" or a "too high value" respectively. On the "Human feed back Device" (9), the RFD may me visualised on e.g. a bar graph array as the centre value.

The displacement is measured by the Detector (6), which detects the signal emanating from the Transmitter (4) passing the optional Aperture (5). One or two of these items, i.e. the Transmitter (4) or the Aperture (5) or the Detector (6) may be located directly on the Vibrating Probe (8) whereas at least one item should be spatially fixed.

The output signal from the detector (6) corresponds to a spatial position (10) of the probe (8). This signal may be processed, e.g. filtered and then converted to a digital signal (DA-conversion) within the Micro Computer System (1). The digital signal is read by a microcomputer, which is a part of the Micro Computer System (1). The microprocessor compares read digital signal caused by the contact force (F) with a previously stored value for the signal caused by the calibrating force (RF) and outputs the difference on the "Human feed back Device" (9).

Instead of maintaining a calibrated static offset for the spatial position (10), the offset may be outbalanced by applying an overlaid calibrated DC-current (i_{c}) in the electrical current signal to the Electro dynamic Vibrator (3). This will create an opposite force (F_{c}), to out-balance the external applied force (F), which will render a zero static offset for the spatial position (10) when the correct static force (F) is applied by the human body part (7).

The calibrated DC-current (i_{c}) may be created within the Micro Computer System (1) where after the signal may be amplified by the Amplifier (2) to control the Electro dynamic Vibrator (3). The system is calibrated by first measuring the spatial position (10) when the system is unloaded, i.e. without any applied contact force (F). Then a "calibration weight" is mounted on the Probe (8) when the system still is unloaded, i.e. no additional force (F) asserted except for the calibration weight. The required DC-current (i_{c}) is automatically adjusted by the Micro Computer System (1) so that zero offset is achieved for the spatial position (10), i.e. when the spatial position (10) is the same as when no "calibration weight" is mounted on the probe (8). At zero offset the applied DC-current (i_{c}) is measured and the value is permanently stored in the Micro Computer System (1).

During normal operation the stored calibrated DC-current (i_{c}) is added to the electrical current signal to the Electro dynamic Vibrator (3). The contact force (F) created by the human body part (7) will cause a static displacement that is measured by the Detector (6), which detects the signal emanating from the Transmitter (4) passing the optional Aperture (5). One or two of these items, i.e. the Transmitter (4) or the Aperture (5) or the Detector (6) may be located directly on the Vibrating Probe (8) whereas at least one item should be spatially fixed.

The contact force (F) is equal to the calibration weight when the measured static displacement for the spatial position (10) is zero. A contact Force (F) below, at or above the calibration weight will then be presented on the "Human feed back Device" (9) as a "too low value", equal or a "too high value" respectively. On the "Human feed back Device" (9), the contact Force (F) may me visualised on e.g. a bar graph LED array

During normal operation, i.e. when the VPT's are recorded the spatial position (10) signal is measured from the Detector (6). This signal may be processed in any way in the Micro Computer System (1), e.g. low pass filtered. The filtered signal from the detector (6) will look something like either as in figure 3 or as in figure 4 below, depending of the selected method for monitoring the static skin force (F) applied by the patient (7). In these figures, x1 corresponds to the spatial position (10) for an unloaded system and x_{cal} to the spatial position (10) when the calibration weight is mounted on the vibrating probe (8) without added DC-current (i_{c}).

The VPT is preferably recorded by reading the real acceleration from an accelerometer sensor mounted directly on the vibrating probe (8). To enhance the accuracy it's also important to register the current skin temperature since the VPT vary with this parameter. The skin temperature may be measured continuously during the measurement or at least at the beginning, i.e. just before start of measurement. The temperature may be measured with e.g. a temperature sensor mounted on the vibrating probe (8) or in a separate place elsewhere on the measuring device.

Prior to a measurement the device shall perform a self-calibration to make sure that the required starting conditions prevails. This calibration shall at least include, a tare of the spatial position (10), a frequency control to make sure that the used frequencies run within certain limits and a measurement of background (vibration) noise. Additionally the maximum and minimum recordable amplitudes and accelerations may be measured during the self-calibration.

The "Human feed back Device" (9) is used to report the measuring status to both the Operator and the Patient to be tested. Prior to a measurement the device (9) should tell when the device is ready (calibration finished). During the measurement the device (9) should show the status for the applied skin contact force (F), i.e. if the force is too high, too low or within required limits. The used "feed back principle" may be e.g. as light by using a LED/lamp-array with different colours, a flashing lamp or a LED with different flashing frequencies or some kind of numerical or graphical display, to represent the status. An audible feedback signal (speaker or headphones) may also be used as a "Human feed back Device" (9) where a combination of different frequencies and/or amplitudes are utilised to represent the status.

As an extra feature it is also possible to automatically compensate the registered VPT if the applied contact skin force (F) is outside the required limits, i.e. when the force is either too high or too low.
For this case, the actual spatial vibration amplitude (mean value), read via the Detector (6), is used to compensate for an erroneous contact force (F). If the applied contact skin force (F) is too low in a balanced system (fig 4) the measured mean value (X) of the spatial position (10) is larger than X1 in figure 4. The offset, X-X1 can then be converted to a specific acceleration offset value, which should be added to the read acceleration in order to get a compensated VPT. The same principle will also work if the applied contact skin force (F) is too high in a balanced system. In that case the read offset value (X-X1) will be negative which corresponds to a negative acceleration offset. The read acceleration should then be reduced with the corresponding converted negative acceleration offset in order to get a compensated VPT.
A full test cycle comprises the following steps:
1. The Operator starts a measurement by e.g. pressing a start button or entering a start command to the device.
2. The device starts with a self-calibration, which is displayed on the "Human feed back Device" (9). When the self-calibration is finished the "Human feed back Device" (9) reports a ready to measure condition.
3. The Patient to be examined applies the appropriate body part, e.g. a finger on the vibrating probe (8) whereas the "Human feed back Device" (9) reports the applied skin force (F). At this stage an integrated temperature sensor on the vibrating probe (8) may measure the skin temperature. Alternatively the temperature may measured in a different manner shortly before the body part is placed on the vibrating probe (8).
4. When the applied skin force is within the required limits, the probe starts to vibrate in a predetermined ascending sequence.
5. When the Patient feels a vibration he/she presses an external button, which will switch the vibration to a descending sequence. During the descending sequence the Patient continue to press the external button until he/she does not feel vibrations any more.
6. When the Patient releases the external button, i.e. when he/she does not feel any vibration, the device will switch back to an ascending sequence and the procedure jumps back to point 5 again and so on, until a full test sequence is completed. A completed test sequence includes changes in the vibration frequencies according to a well-defined scheme.
7. The vibration excitation stops when the VPT's has been registered for all required frequencies. The recorded VPT's may then be compared with normative data from healthy persons. The result may be reported to the Operator as e.g. a SI-value which is an absolute figure telling if the Patient is healthy or not, i.e. in terms of neuropathy.

During the test cycle according to point 5 and 6 above the applied skin contact force is monitored continuously by reading the spatial position (10). The read spatial position (10) is converted to a contact force (F), which is continuously displayed on the "Human feed back Device" (9). The Patient reads the output and adjusts the contact force (F) accordingly. The device may calculate an internal compensation to adjust the recorded VPT if the Patient does not make any adjustments or if made adjustments are insufficient. The VPT's are recorded as the mean value of the read max and min acceleration (rms values) during the ascending and descending cycle.

### Unbalanced system:

When the correct contact force (F) is applied by the patient (7), the offset for the dc-component in the spatial position (10) signal shall be equal to (x₁ - x_{cal}). If the measured offset is higher then the patient must decrease the applied skin-force (F) and vice versa, i.e. increase the applied skin force (F) if the offset is to low. With this method no added DC-current component (i_{c}) is necessary in the electrical signal, which drives the Electro dynamic Vibrator (3).

### Balanced system:

A dc-current (i_{c}) is added to the electrical signal, which drives the Electro dynamic Vibrator (3). When this current (i_{c}) is added and when the correct contact force (F) is applied by the patient (7), the dc-component in the spatial position (10) signal shall be equal to x₁, which corresponds to a zero static offset. If the measured spatial position (10) is less than x₁ then the patient must decrease the applied skin-force (F) vice versa, i.e. increase the applied skin force (F) the spatial position (10) is larger than x₁.

### Spatial detection

The spatial position (10) can be measured in many ways, but the basic principle is that the vibrating probe (8) is moved when the human body part (7) applies a force (F) on the probe (8). The spatial position (10) and the subsequent movement will alter the signal from the transmitter (4) and the detector (6) measures this spatial alteration of the transmitted signal. In this respect the transmitter (4) can be mounted directly on the vibrating probe (8) while the detector (6) is fixed in space. Alternatively the detector (6) may be mounted directly on the vibrating probe (8) while the transmitter (4) is fixed in space. As a second alternative both the transmitter (4) and the detector (6) are fixed in space whereas the aperture (5) is mounted directly on the vibrating probe (8). The combination of the transmitter (4) and the detector (6) makes a matched pair that can use different techniques and some examples are:

| **Transmitter (4)** | **Detector (6)** |
|---|---|
| Light Emitting Diode, LED | Position Sensitive Detector (PSD) |
| LASER Diode | Position Sensitive Detector (PSD) |
| Light Emitting Diode, LED | Photo detector |
| LASER Diode | Photo detector |
| Permanent Magnet | Magnetic field sensor |
| Electro Magnet | Magnetic field sensor |

## Claims

1. An apparatus for testing or screening of peripheral neuropathies at a skin site (7) of a subject comprising:
vibration generating means (3) having a contact element (8) for positioning at said skin site (7) and being adapted to vibrate at specific frequencies,
frequency generating means (1) connected to said vibration generating (3) for supplying a known discrete frequency signal to said vibration generating means (3),
control circuit means adapted to modify an amplitude of said known discrete frequency signal in an ascending and descending mode,
switch means actuable by said subject to provide response signals to said control circuit means as a response to an ascending and a descending amplitude of said known frequency signal,
said control circuit means having processing means for obtaining a mean threshold signal value from said response signals;
wherein
said vibration generating means (3) is an electrodynamic vibrator (3) adapted to move the contact element (8) when a current is applied to it; and
said frequency generating means (1) is a microcomputer system (1) comprising interface electronics and adapted to drive the electrodynamic vibrator (3);
and wherein
the apparatus further comprises contact element position determination means comprising a transmitter (4) and a detector (6) wherein the output signal from the detector (6) depends on the spatial position of the contact element(8); and
means for controlling and monitoring the static and dynamic contact force (F) between said skin site (7) and said vibrating generating means (8) in such a manner that said contact force (F) is within a predefined range; **characterised in that**
said means for controlling and monitoring the static and dynamic contact force (F) are implemented by means of the microcomputer system (1) being configured:
to determine an offset between the detected spatial position of the contact element due to the contact force on said contact element and a predefined reference position corresponding to the predefined range for the contact force; and to
automatically adjust a DC-current (ic) overlaid to the signal driving the electrodynamic vibrator (3), such that it produces an opposing probe force (Fc) so that a zero offset is achieved for the spatial position.

2. An apparatus according to claim 1 wherein said transmitter (4) is arranged to operate either by light emitting or by transmitting an electromagnetic field and wherein said detector (6) is arranged to operate either by photo electric detection or by magnetic field sensing.

3. An apparatus according to claims 1-2 wherein the means for keeping continuous contact force between said skin site and said vibrating means is automated by a feedback force compensation unit.

4. An apparatus according to claims 1-3 wherein said mean threshold signal value is a vibrotactile perception threshold that is recorded by reading the real acceleration from an accelerometer.

5. An apparatus according to claims 1-4 comprising a human feedback device (9) adapted to use a feed back principle based on light; such as LED or lamp arrays; flashing LED's or lights or a graphical or numerical display.

6. An apparatus according to claims 1-5, comprising a temperature measuring device arranged for measuring a temperature at said skin site prior and during the entire test.

7. A method for identifying vibrotactile perception thresholds at a skin site of a subject comprising employing an apparatus according to any of claims 1-6 for such identification.

## Patentansprüche

1. Gerät zur Untersuchung von oder Vorsorgeuntersuchung auf periphere Neuropathien an einer Hautstelle (7) einer Testperson, umfassend:
Mittel zur Erzeugung von Vibrationen (3), das ein Kontaktelement (8) zur Positionierung an der Hautstelle (7) aufweist, und das so angepasst ist, dass es bei bestimmten Frequenzen vibriert,
Mittel zur Erzeugung von Frequenzen (1), das mit dem Mittel zur Erzeugung von Vibrationen (3) verbunden ist, so dass ein bekanntes diskretes Frequenzsignal an das Mittel zur Erzeugung von Vibrationen (3) geliefert wird,
Regelkreismittel, das so angepasst ist, dass es eine Amplitude des bekannten diskreten Frequenzsignals in einem aufsteigenden und einem absteigenden Modus modifiziert,
Schaltmittel, das sich von der Testperson betätigen lässt, um Antwortsignale an das Regelkreismittel als eine Antwort auf eine aufsteigende und eine absteigende Amplitude des bekannten Frequenzsignals zu liefern,
wobei das Regelkreismittel ein Verarbeitungsmittel zum Gewinnen eines durchschnittlichen Schwellensignalwerts aus den Antwortsignalen aufweist,
wobei es sich bei dem Mittel zur Erzeugung von Vibrationen (3) um ein elektrodynamisches Vibrationsgerät (3) handelt, das so angepasst ist, dass es das Kontaktelement (8) in Bewegung versetzt, wenn es unter einen Strom gesetzt wird; und
es sich bei dem Mittel zur Erzeugung von Frequenzen (1) um ein Mikrocomputersystem (1) handelt, das eine Schnittstellenelektronik umfasst und so angepasst ist, dass es das elektrodynamische Vibrationsgerät (3) antreibt; und wobei
das Gerät ferner Mittel zur Positionsbestimmung des Kontaktelements umfasst, die einen Sender (4) und einen Detektor (6) umfassen, wobei das Ausgangssignal von dem Detektor (6) von der räumlichen Position des Kontaktelements (8) abhängt; und
Mittel zur Steuerung und Überwachung der statischen und dynamischen Kontaktkraft (F) zwischen der Hautstelle (7) und dem Mittel zur Erzeugung von Vibrationen (8) auf solch eine Weise, dass sich die Kontaktkraft (F) innerhalb eines vorbestimmten Bereiches befindet;
**dadurch gekennzeichnet, dass** die Mittel zur Steuerung und Überwachung der statischen und dynamischen Kontaktkraft (F) dadurch implementiert werden, dass das Mikrocomputersystem (1) derart konfiguriert wird, dass es:
eine Verschiebung zwischen der erkannten räumlichen Position des Kontaktelements, welche aufgrund der auf das Kontaktelement ausgeübten Kontaktkraft vorliegt, und einer vordefinierten Referenzposition, welche dem vordefinierten Bereich für die Kontaktkraft entspricht, bestimmt; und dass es
einen Gleichstrom (ic) automatisch anpasst, welcher dem Signal überlagert ist, durch welches das elektrodynamische Vibrationsgerät (3) angetrieben wird, wodurch eine entgegengesetzte Testkraft (Fc) erzeugt wird, so dass für die räumliche Position eine Verschiebung von Null erreicht wird.

2. Gerät gemäß Anspruch 1, wobei der Sender (4) so eingerichtet ist, dass er entweder durch Lichtemission oder durch die Übertragung eines elektromagnetischen Feldes betrieben wird, und wobei der Detektor (6) so eingerichtet ist, dass er entweder durch fotoelektrische Erkennung oder durch Magnetfeldsensortechnik betrieben wird.

3. Gerät gemäß Ansprüchen 1 bis 2, wobei das Mittel zur Aufrechterhaltung einer kontinuierlichen Kontaktkraft zwischen der Hautstelle und dem Vibrationsmittel durch eine Feedback-Kraftkompensationseinheit automatisiert ist.

4. Gerät gemäß Ansprüchen 1 bis 3, wobei es sich bei dem durchschnittlichen Schwellensignalwert um eine vibrotaktile Wahrnehmungsschwelle handelt, welche durch das Ablesen der tatsächlichen Beschleunigung von einem Beschleunigungsmesser aufgezeichnet wird.

5. Gerät gemäß Ansprüchen 1 bis 4, eine menschliche Feedback-Vorrichtung (9) umfassend, die so angepasst ist, dass sie ein auf Licht beruhendes Feedback-System benutzt; wie beispielsweise LED oder Lampen-Arrays; blinkende LEDs oder Lichter oder ein graphisches oder numerisches Display.

6. Gerät gemäß Ansprüchen 1 bis 5, eine Temperaturmessvorrichtung umfassend, welche so eingerichtet ist, dass sie eine Temperatur an der Hautstelle vor und während des gesamten Tests misst.

7. Verfahren zur Identifizierung von vibrotaktilen Wahrnehmungsschwellen an der Hautstelle einer Testperson, die Verwendung eines Geräts gemäß einem beliebigen der Ansprüche 1 bis 6 zum Zwecke solch einer Identifizierung umfassend.

## Revendications

1. Appareil pour tester ou dépister des neuropathies périphériques au niveau d'un site de la peau (7) d'un patient comprenant :
un moyen de génération de vibrations (3) ayant un élément de contact (8) pour un positionnement au niveau dudit site de la peau (7) et étant destiné à vibrer à des fréquences spécifiques,
un moyen de génération de fréquence (1) connecté audit moyen de génération de vibrations (3) pour fournir un signal à fréquence discrète connue audit moyen de génération de vibrations (3),
un moyen de circuit de commande destiné à modifier une amplitude dudit signal à fréquence discrète connue dans un mode croissant et décroissant,
un moyen de basculement pouvant être actionné par ledit patient pour fournir des signaux de réponse audit moyen de circuit de commande comme une réponse à une amplitude croissante et décroissante dudit signal à fréquence connue,
ledit moyen de circuit de commande ayant un moyen de traitement destiné à obtenir une valeur de signal de seuil moyenne à partir desdits signaux de réponse ;
dans lequel
ledit moyen de génération de vibrations (3) est un vibrateur électrodynamique (3) destiné à déplacer l'élément de contact (8) lorsqu'un courant lui est appliqué ; et
ledit moyen de génération de fréquence (1) est un système de micro-ordinateur (1) comprenant des circuits électroniques d'interface et destiné à attaquer le vibrateur électrodynamique (3) ;
et dans lequel
l'appareil comprend en outre un moyen de détermination de position d'élément de contact comprenant un émetteur (4) et un détecteur (6) dans lequel le signal de sortie provenant du détecteur (6) dépend de la position spatiale de l'élément de contact (8) ; et
un moyen destiné à commander et à surveiller la force de contact statique et dynamique (F) entre ledit site de la peau (7) et ledit moyen de génération de vibration (8) d'une manière telle que ladite force de contact (F) se trouve dans une plage prédéfinie ;
**caractérisé en ce que** ledit moyen destiné à commander et à surveiller la force de contact statique et dynamique (F) est réalisé au moyen du système de micro-ordinateur (1) qui est configuré pour :
déterminer un décalage entre la position spatiale détectée de l'élément de contact due à la force de contact sur ledit élément de contact et une position de référence prédéfinie correspondant à la plage prédéfinie pour la force de contact ; et
ajuster automatiquement un courant continu (ic) superposé au signal attaquant le vibrateur électrodynamique (3), de sorte qu'il produise une force de sonde opposée (Fc) de sorte qu'un décalage nul soit obtenu pour la position spatiale.

2. Appareil selon la revendication 1 dans lequel ledit émetteur (4) est conçu pour fonctionner soit en émettant de la lumière soit en transmettant un champ électromagnétique et dans lequel ledit détecteur (6) est conçu pour fonctionner soit par détection photoélectrique soit par détection de champ magnétique.

3. Appareil selon les revendications 1 à 2 dans lequel le moyen pour conserver une force de contact continue entre ledit site de la peau et ledit moyen de vibration est automatisé par une unité de compensation de force à rétroaction.

4. Appareil selon les revendications 1 à 3 dans lequel ladite valeur du signal de seuil moyenne est un seuil de perception vibro-tactile qui est enregistré par une lecture de l'accélération réelle à partir d'un accéléromètre.

5. Appareil selon les revendications 1 à 4 comprenant un dispositif de rétroaction humain (9) destiné à utiliser un principe de rétroaction basé sur la lumière ; par exemple des réseaux de diodes DEL ou de lampes ; des diodes DEL ou des lumières clignotantes ou un affichage graphique ou numérique.

6. Appareil selon les revendications 1 à 5, comprenant un dispositif de mesure de température conçu pour mesurer une température au niveau dudit site de la peau avant et durant le test entier.

7. Procédé destiné à identifier des seuils de perception vibro-tactile au niveau d'un site de la peau d'un patient comprenant l'emploi d'un appareil selon l'une quelconque des revendications 1 à 6 pour une telle identification.
